# EUROPEAN PATENT SPECIFICATION

(11) **EP 1 621 130 B1**
(45) Date of publication and mention of the grant of the patent: **03.10.2018**
(21) Application number: 04724836.4
(22) Date of filing: 31.03.2004
(51) Int. Cl.: A61B 5/08, A61B 5/0468, A61M 16/10

(54) **SLEEP RESPIRATORY DISORDER EXAMINATION DEVICE AND TREATMENT SYSTEM**
UNTERSUCHUNGSVORRICHTUNG FÜR SCHLAFATEMSTÖRUNGEN UND BEHANDLUNGSSYSTEM
DISPOSITIF D'EXAMEN DES TROUBLES RESPIRATOIRES DU SOMMEIL ET SYSTEME DE TRAITEMENT DESDITS TROUBLES

(30) Priority: 02.04.2003 JP 2003098992
(43) Date of publication of application: 01.02.2006
(73) Proprietor: Teijin Pharma Limited, Tokyo 100-0011 (JP)
(72) Inventor: IMOSE, Kazuo, Tokyo 100-0011 (JP)
(74) Representative: Hoffmann Eitle
(86) International application number: PCT/JP2004/004712
(87) International publication number: WO 2004/089212

(56) References cited:
- WO-A-96/32055
- JP-A- 11 504 840
- JP-A- 2001 505 441
- JP-A- 2003 000 559
- JP-A- 2004 159 888
- JP-B- 6 028 653
- US-A- 6 120 441
- US-B1- 6 527 729
- TATEISHI, O.: 'Cheyne-Stokes Kokyu Gappeirei no Shinpaku Hendo' JAPANESE JOURNAL OF ELECTRO CARDIOLOGY vol. 22, no. 1, 25 March 2002, pages 82 - 84, XP002984666

## Description

The present invention relates to an examination apparatus and a therapeutic system, for use in carrying out oxygen therapies in which an oxygen-enriched gas is supplied for respiration of a patient in order to calm a sleep respiratory disturbance that is a complication symptom of chronic heart failure.

WO 96/32055 describes how the occurrence of an arousal in a patient associated with an apneic or hypopneic episode can be determined. Sensors are placed on a patient to obtain signals representative of at least two physiological variables, for example skin conductance, heart rate and blood oxygen concentration. The signals are conditioned by conditioning circuitry, then processed by a processor to correlate at least two thereof. A coincident change in at least two of the processed signals is indicative of the occurrence of an arousal, that in turn indicates an apneic or hypopneic episode has occurred. A patient thus can be diagnosed as suffering conditions such as obstructive sleep apnea.

US-A-6 120 441 describes an apparatus and a method for the stationary and ambulant detection, recording and quantitative analysis of sleep disorders, sleep-related respiratory disturbances, cardiac rhythm disturbances, myoclonia, variations in blood pressure, depth of sleep parameters, movement parameters and disorder parameters for the quality control of diagnoses. Various sensors detect a patient's body functions which are stored in a recorder. The stored data are then transferred to a computer where they are analyzed and evaluated.

US 6,527,729 B1 describes a method for monitoring the progression of the disease of a heart failure patient. An implantable or other ambulatory monitor senses acoustic signals including heart and lung sounds within the patient. Significant changes in the energy content of either the heart or lung sounds is indicative of a heart failure exacerbation. This information may be used to warn the patient or healthcare providers of changes in the patient's condition warranting attention.

### Summary of home oxygen therapy

Conventionally, oxygen therapies in which patients suffering from a respiratory disease are supplied from an oxygen cylinder have been carried out, and recently, apparatuses for supplying a gas for respiration in an attempt to obtain an oxygen-enriched gas by separation and concentration of oxygen in the air was developed. Accordingly, oxygen therapies in which such a gas is used have gradually prevailed.

Such an oxygen therapy may be carried out while the patient is hospitalized to a medical institution. However, in cases where the patient has developed a chronic symptom of the respiratory disease, and calming and stabilization of the symptom must be attempted for a long period of time by carrying out this oxygen therapy, a therapeutic method is carried out in which a supplying apparatus of a gas for respiration is installed at the patient's home; an oxygen-enriched gas supplied by this supplying apparatus of a gas for respiration is introduced to the vicinity of nasal cavity of the patient using a tubing member referred to as cannula; and the patient inhales the gas. This type of oxygen therapy is also referred to as "home oxygen therapy (HOT)".

The home oxygen therapies have been carried out since coverage by medical insurance in 1985, which have been prescribed predominantly for chronic obstructive pulmonary disease (COPD) and post lung tuberculosis sequelae. Approximately estimated number of the patients in Japan reaches to about eighty thousand in 2000, which corresponds to 60 to 65 per hundred thousand of population. In addition, improvement of vital prognosis of patients by this home oxygen therapy was also reported by former Ministry of Health and Welfare, surveillance study group of respiratory failures as specified diseases , and the like. One of the grounds for such a therapeutic effect exerted by the home oxygen therapy on COPD is speculated to involve effectiveness of oxygen inhalation in preventing advance in the COPD patient from the state of impossible sufficient respiration which persists for a long period of time, to "right ventricular failure" caused by increased strain on right ventricle, which supplies blood to lung, leading to cardiac hypertrophy and deteriorated function.

Process of introduction of the home oxygen therapy on a patient followed by sustaining the therapy will be explained serially.

First, a patient suffering from a respiratory disease visits a medical institution and has an inspection by the doctor. When the doctor assesses that this patient requires the home oxygen therapy as a result of the inspection, the doctor conducts introduction for receiving a home oxygen therapy on the patient, and initial instruction in medical aspects. The introduction and instruction may be conducted while hospitalization in this medical institution for specified days, alternatively, may be conducted after serving introduction to other medical institution, for example, a core foundation hospital in the district, and hospitalization in this foundation hospital.

When course of the disease in the patient is favorable as a result of the introduction and initial medical instruction described above, attending physician issues an instruction describing a prescription for carrying out a home oxygen therapy on this patient. According to the issued instruction, the supplier of the supplying apparatus of a gas for respiration who has previously concluded a contract with this medical institution carries the supplying apparatus of a gas for respiration according to the prescription to the patient' s home, and installation of the supplying apparatus of a gas for respiration and setting of various conditions such as oxygen concentration, flow rate of the gas, and the like are additionally performed so that this patient can receive the home oxygen therapy appropriately on the basis of the prescription.

When the preparation is completed according to the above procedures, the patient is permitted to consecutively receive the home oxygen therapy to inhale an oxygen-enriched gas supplied by thus installed supplying apparatus of a gas for respiration at home. For coverage by medical insurance of this home oxygen therapy, inspection by the doctor is required at the hospital as an outpatient or at home once per month, without fail.

In addition to the chronic obstructive pulmonary disease (COPD), post lung tuberculosis sequelae and the like, chronic heart failures (hereinafter, may be also referred to as CHF) has been suggested as diseases to which the home oxygen therapy as described above is to be applied. Grounds for trying to apply the home oxygen therapy for this CHF predominantly involve attempts to alleviate symptoms in CHF patients by ameliorating a Cheyne-Stokes respiratory symptom (to be described later, may be also referred to as Cheyne-Stokes respiration) that are often manifested in CHF. Hereinafter, effects of a therapeutic method in which a chronic heart failure (CHF) patient is subjected to an oxygen therapy will be explained based on known documents.

### Effect of oxygen therapy on chronic heart failure

### (1) Preface

Chronic heart failure (CHF) is defined as a syndrome which leads to peripheral circulatory failure, and exacerbation of exercise tolerance, QOL (Quality Of Life) and life expectancy which may result from chronically compromised left ventricular function. Therapeutic objects of chronic heart failure include suppression of advance of cardiac function disorders (prevention of acute exacerbation), and amelioration of subjective symptoms, exercise tolerance, QOL and life expectancy.

Principal therapeutic method for CHF involves pharmacotherapeutics of predominantly using a diuretic drug, an ACE inhibitor (a drug for avoiding lack of a substance to act on kidney to dilate blood vessels, thereby accompanying lowering of blood pressure (bradykinin), an angiotensin converting enzyme inhibitor), a *β*-blocker (a sympatholytic agent that blocks *β* receptor (adrenergic blocking agent)) or the like, and control of routine life style such as dietary instructions and education of the patient.

Oxygen therapy is referred to as being effective in improvement of arterial oxygen saturation and lowering of pulmonary vascular resistance. In hospitalization due to acute exacerbation of a chronic heart failure, oxygen therapy has been carried out.

### (2) Respiratory state at night and prognosis in CHF

In recent years, it is referred to that 40% of CHF is accompanied by a complication of a Cheyne-Stokes respiratory symptom (may be also referred to as CSR: Cheyne-Stokes respiration; symptoms in which gradual increase and gradual decrease in respiratory airflow and successively occurring apnea or hypopnea of central type are repeated.). As a result of follow-up researches on patients suffering from a complication of a Cheyne-Stokes respiratory symptom and on patients not suffering from a complication of a Cheyne-Stokes respiratory symptom, one fatal case was found in 7 cases without complication of a Cheyne-Stokes respiratory symptom, while 5 fetal cases and two cases of receiving heart transplantation (cardiac death) were found in 9 cases with a complication of a Cheyne-Stokes respiratory symptom. Thus, it was suggested that prognoses are unfavorable in patients having a complication of a Cheyne-Stokes respiratory symptom in comparison with cases without complication of a Cheyne-Stokes respiratory symptom (Increased Mortality Associated with Cheyne-Strokes Respiration in Patients with Congestive Heart, Hally PJ & Zuberi-Khokhar NS: Am J Respir Crit Care Med, Vol 153, 272-276, 1996).

### (3) Effect of oxygen therapy on CHF

A Cheyne-Stokes respiratory symptom is often observed in CHF, accompanied by nocturnal hypoxia and sleep disorder due to wakefulness. Nocturnal hypoxia and wakefulness account for increase in pulmonary artery pressure and sympathetic nerve activities thereby resulting in reduction of exercise toleranc

Oxygen therapies are effective in amelioration of the Cheyne-Stokes respiratory symptom, and expected to improve exercise tolerance in CHF patients with a complication of a Cheyne-Stokes respiratory symptom. As a result of comparison of PSG (Polysomnography) examination (described later), exercise stress test, observation of cardiac failure symptoms and the like through oxygen therapy and air inhalation for each one week in 22 CHF patients under random, crossover, DBT (Double Blind Test) conditions, it was found that a Cheyne-Stokes respiratory symptom was ameliorated, and maximum oxygen uptake to be a marker of exercise tolerance was improved by a nocturnal oxygen therapy. Significant amelioration of daytime cardiac failure symptoms was not found (Improvement of Exercise Capacity With Treatment of Cheyne-Stokes Respiration in Patients With Congestive Heart Failure, Andreas S et al. : JACC, Vol 27 (6), 1486-90, 1996).

Cheyne-Stokes respiratory symptoms disturb sleep, and cause daytime drowsiness and dysgnosia. In addition, Cheyne-Stokes respiratory symptoms are independent factor of prognosis. Results of PSG examination, examination of catecholamine in urine as a marker of sympathetic nerve activity, and comparison in both cases of oxygen therapy and air inhalation each four weeks in 11 CHF patients under random, crossover, DBT conditions showed that nocturnal oxygen therapy ameliorated the Cheyne-Stokes respiratory symptom, and lowered the amount of noradrenaline in urine. Significant amelioration of daytime cardiac failure symptoms was not observed (Effect of Oxygen on Sleep Quality, Cognitive Function and Sympathetic Activity in Patients With Chronic Heart Failure and Cheyne-Stokes Respiration, Staniforth AD et al. : Eur Heart J, Vol 19, 922-928, 1998).

A home oxygen therapy was introduced to CHF patients, and comparison was made between before and one month following the introduction in respect of minimum amount of exercise to be aware of exertional dyspneic feeling based on interview on SAS (Specific Activity scale), and between hospitalization frequencies due to exacerbation of cardiac failure before the introduction and in one year following the introduction of the home oxygen therapy. Consequently, it was found that the home oxygen therapy improved SAS from 2.5 ± 0.9 before the introduction to 3.3 ± 1.0 METs in one month following the introduction of the home oxygen therapy, and that hospitalization frequency was significantly decreased from 1.2 ± 1.3 times before the introduction to 0.8 ± 1.2 time after the introduction for one year (Effects of Home Oxygen Therapy on Patients With Chronic Heart Failure, R. Kojima, M. Nakatani, et al.: JAC, Vol 38, 81-86, 2001).

As in the foregoings, ratio of a complication of a Cheyne-Stokes respiratory symptom in CHF is so high, and the Cheyne-Stokes respiratory symptom causes nocturnal hypoxia and sleep disorder due to wakefulness, thereby resulting in decrease of exercise tolerance in CHF patients. On the other hand, oxygen therapies are effective in amelioration of a Cheyne-Stokes respiratory symptom, and can ameliorate exercise tolerance in CHF patients with a complication of a Cheyne-Stokes respiratory symptom.

Furthermore, performing home oxygen therapy to carry out the oxygen therapy at home enables continuation of oxygen therapy for a long period of time under fewer economical and social burdens without need of hospitalization. Thus, amelioration of a Cheyne-Stokes respiratory symptom described above and amelioration of exercise tolerance accompanied thereby in CHF patients can be more certainly executed with fewer burdens.

### Conventional method of selecting a patient - PSG

As described hereinabove, it has been known that carrying out an oxygen therapy, particularly performing a home oxygen therapy on CHF patients is effective. However, there have been problems in conventional technical constitutions which are difficult in solving, respectively, i.e., "Operation of selecting a patient for whom an oxygen therapy is effective among CHF patients can not be readily and certainly conducted."; and "Therapeutic effects achieved when an oxygen therapy is carried out on a CHF patient can not be readily and certainly ascertained.", which will be explained below.

Conventionally, in order to select a patient for whom an oxygen therapy is effective among CHF patients, sleep examination as described below in which an apparatus referred to as "PSG (Polysomnography: polysomnographic apparatus)" is used (hereinafter, this sleep examination is referred to as "PSG" or "PSG examination") has been generally performed, thereby determining presence/absence of a Cheyne-Stokes respiratory symptom in a CHF patient to select a patient having a Cheyne-Stokes respiratory symptom as a patient to whom an oxygen therapy should be carried out.

The PSG is an examination for quantitatively determining deepness of sleep (stage of sleep), fragmentation of sleep, presence/absence of arousal response, construction of sleep, efficiency of sleep and the like as well as details of respiratory state, by determining, in addition to basic items such as respiratory airflow, sound of snore and arterial oxygen saturation (SpO₂), more detailed biological information such as electroencephalogram, electromyogram and movement of eyeballs.

In order to perform PSG, the patient is, in many cases, hospitalized to a medical institution or a dedicated facility for the examination referred to as sleep laboratory with the schedule of three days and two nights (performing PSG at first night, and determining a prescription on the second) and sleeps while wearing various sensors attached to an examination instrument referred to as determination and recording apparatus of polysomnography, at each body part of the patient. Then, output signal from each sensor is continuously recorded on a predetermined recording medium (hard disk of a personal computer, memory card or the like) during sleeping.

Data obtained following the recording are analyzed manually through directly analyzing the examination data by the medical .service worker, or using a dedicated apparatus referred to as an automatic polysomnography analysis apparatus. In cases of the automatic analysis, a report of summary of evaluation on multiple items is automatically produced. Examples of the multiple evaluation items include e.g., each item presented in Table 1.

**Table 1 Examples of determination and items in PSG**

| Determination items | Evaluation items |
|---|---|
| Electroencephalogram | Type and depth of sleep, wakefulness |
| Movement of eyeballs | Presence/absence of REM sleep |
| Mentalis muscle electromyogram | Presence/absence of REM sleep |
| Respiration (thermistor) | Presence/absence of airflow through mouth and nose |
| Ventilation exercise | Detection of ventilation exercise in chest and abdominis |
| Electrocardiogram | Arrhythmia or change in heart rate |
| Arterial oxygen saturation | Grasp of hypoxemia |
| Body position | Occurrence frequency of apnea being liable to increase in dorsal position |
| Lower extremity electromyogram | Presence/absence of restless leg syndrome |

The medical service worker can find manifestation of a Cheyne-Stokes respiratory symptom based on description in the report obtained by performing the PSG in light of, for example, following respects. More specifically, when gradual increase and gradual decrease in respiratory airflow and efforts for respiration appeared repeatedly during Stages 1 to 2 (restless sleep), manifestation of a Cheyne-Stokes respiratory symptom will be suspected.

As described above, when PSG is used, the medical service worker can know whether or not manifestation of a Cheyne-Stokes respiratory symptom was present in a subject patient during sleep, and can instruct to carry out an oxygen therapy on the patients found to have a Cheyne-Stokes respiratory symptom as needed, based on the results of this knowledge.

However, according to the method of selecting a patient in which presence/absence of a Cheyne-Stokes respiratory symptom is determined using the PSG, because it is an examination method performed using a large scale of facility for the examination while the patient being admitted in a hospital, management and practice of the examination become an extensive business which can not be readily performed in implementation side of the examination such as a medical institution, while the hospitalization becomes a great burden for the patient side, which also hampers easy implementation.

Accordingly, because the sleep examination in which PSG is used requires performing the examination while wearing numerous sensors at each body part of a patient, wearing operation of the sensors and ascertaining operation thereof, as well as ascertaining operation by an expert examination engineer during recording, and in addition, a large scale determination equipment is required so that the numerous items can be recorded. Thus, hospitalization into a medical institution having these equipments and an expert examination engineer is necessary.

Therefore, requirement of hospitalization upon performing the examination leads to a burden on the patient, which may cause the patient to hesitate the consultation of the examination, thereby lessening the opportunity of consultation of the examination, and opportunity of the therapy.

Moreover, for undertaking practice and management of PSG, various equipments including a determination and recording apparatus of polysomnography constructed so that numerous determination items can be recorded, and an automatic polysomnography analysis apparatus for analyzing those numerous determination items as well as various equipments needed for hospitalization of the patients must be provided. In addition, an examination engineer who attends to wearing of each kind of sensor to the patient must be employed. Accordingly, a great burden has been posed to an administrative person of the examination for installation of the equipments for the examination and management of the examination.

Therefore, in conjunction with no prospect for many patients who use the system due to a great burden of consultation of the examination for the patients, significant difficulty was posed in performing the examination and management of PSG.

In fact, there is described in a home page provided on Internet to allow general public to be accessible <http://nsleep.com/hp/sleep/sl-test/sl-psg.htm> (under the name of medical corporation HSR, Nakamura Clinic), in addition to an explanation of the PSG examination, that "Examination is performed for about 8 hours from around 9 pm to around 6 am. Preparation will be started at half past eight pm for the person who initiates first. At present, there are eight rooms for the examination, and eight persons are examined per day on 5 days in a week on Monday, Tuesday, Wednesday, Thursday and Friday. During the time period, an examination staff makes observation constantly with a monitor. This examination poses great burdens such as time and labor on examining facility, therefore, only limited facilities over the country perform this examination. Because long time is consumed in examination as well as analysis, about 2 weeks will be required until the results can be reported.".

As described above, significant difficulties are involved in performing the examination of PSG and management in a facility such as a medical institution in which PSG can be performed or a sleep laboratory. As a consequence, number of facilities where patients can be utilized is limited, and further, PSG is an examination also used in proving manifestation of obstructive sleep apnea syndromes which have attracted attention recently. Therefore, under current circumstances, many patients have been waiting with reservation for consultation of the examination.

Hence, even though a medical service worker intends the PSG examination to perform on a CHF patient, it can be actually performed later on the reserved date after waiting. Thus, rapid examination and therapy could not have been performed. Additionally, number of facilities in which PSG examination can be performed is limited, for example, number of facilities in one prefecture may be limited only one or two. Therefore, there may be a case in which a patient living far from these facilities can not receive examination.

Under the circumstances described above, in place of the large scale examination method, i.e., PSG, smaller scale examination for narrowed down examination items can be readily supposed, for example, an examination method in which presence/absence of Cheyne-Stokes respiration is determined by observing gradual increase and gradual decrease of respiratory airflow in a patient during sleeping, thereby intending decision for necessity to carry out an oxygen therapy. However, according to such a simplified examination for narrowed down examination items to respiratory airflow and the like, it was difficult to certainly discriminate the presence/absence of a Cheyne-Stokes respiratory symptom, and to decide for necessity to carry out an oxygen therapy on the ground that the boundary region to discriminate the presence/absence of a Cheyne-Stokes respiratory symptom is unclear, and the like when attention is focused on respiratory airflow alone.

### Method of ascertaining therapeutic effect in conventional oxygen therapy

Furthermore, in addition to the examination method for finding the patients, it was conventionally difficult to ascertain a therapeutic effect readily and certainly when an oxygen therapy was carried out on a CHF patient.

More specifically, for observing the state of a patient to ascertain the expected therapeutic effect of the oxygen therapy, the aforementioned PSG and an examination of catecholamine in urine must be conducted while sleeping at night through hospitalization in a medical institution or the like. Thus, as explained previously, it poses significant burdens on the patient and medical institution. In addition, according to a simplified examination focused only on respiratory airflow of the patient as described above, ascertainment could not be conducted similarly.

Hence, it was conventionally very difficult to conduct an operation to select a patient for whom an oxygen therapy is effective among CHF patients, and an operation to ascertain a therapeutic effect of the oxygen therapy carried out on thus selected patient without performing PSG for ascertaining a Cheyne-Stokes respiratory symptom, and without hospitalization, i.e., at home using a simple equipment. As a matter of course, any method of solving the problems described above was not shown in any one of the aforementioned documents.

The present invention was made taking into account of the situation described above, and an object of the invention is to provide an examination apparatus and a therapeutic system for e.g., reliably selecting a patient for whom an oxygen therapy is effective among CHF (chronic heart failure) patients, ascertaining a therapeutic effect of the oxygen therapy carried out on thus selected patient without fail, and performing these operations without hospitalization using a simple equipment at home.

According to a first aspect of the invention, there is provided an examination apparatus for use in selecting a patient for whom an oxygen therapy is effective among patients having a sleep respiratory disturbance, the apparatus comprising: a sensor for detecting presence/absence or magnitude of respiratory airflow of the subject patient; a unit for determining an electrocardiogram waveform of the subject patient, and an analysis unit for analysing the enhanced state of sympathetic nerve based on the determined electrocardiogram waveform with a heart rate variability analytical procedure; an analysis unit for analysing synchronization of transition of the respiratory state into a Cheyne-Stokes respiratory symptom, in which apnea and respiratory states are repeated, with transition of enhancement of sympathetic nerve into an abnormal state and for producing graphs showing (A) said transition of respiratory airflow in conjunction with (B) said transition of enhanced state of sympathetic nerve, of the subject patient during sleeping; and an output part for displaying or printing said graphs.

According to a second aspect of the invention, there is provided a therapeutic system which comprises (1) an examination apparatus according to the first aspect set out above and (2) a supplying apparatus of an oxygen-enriched gas for respiration for the purpose of carrying out the oxygen therapy.

For a better understanding of the invention and to show how the same may be carried into effect, reference will now be made, by way of example, to the accompanying drawings, in which:
Fig. 1 is a constitution diagram illustrating a biological information monitoring apparatus carried by a therapeutic system that is a preferable Example according to an embodiment of the present invention;
Fig. 2 is a schematic flow diagram illustrating a supplying apparatus of an oxygen-enriched gas carried by the therapeutic system of this Example;
Fig. 3 is a flow chart illustrating procedures for carrying out a therapy using the therapeutic system of this Example; and
Fig. 4 is a schematic view illustrating results of determination and analysis obtained using the biological information monitoring apparatus shown in Fig. 1.

The following describes an examination apparatus, a therapeutic system, a selection method and a therapeutic method having each construction described in the following 1) to 13).
1) An examination apparatus for use in selecting a patient for whom an oxygen therapy is effective among patients having a sleep respiratory disturbance, the apparatus having an output part for displaying or printing both of: (A) transition of respiratory airflow; and (B) transition of enhanced state of sympathetic nerve, of the subject patient during sleeping.
2) The examination apparatus according to the above item 1) which comprises a unit for determining an electrocardiogram of the subject patient, and an analysis unit for analyzing the enhanced state of sympathetic nerve based on the determined electrocardiogram wave form with a heart rate variability analytical procedure.
3) The examination apparatus according to the above item 2) which comprises a sensor for detecting presence/absence or magnitude of respiratory airflow of the subject patient, and an analysis unit for analyzing synchronization of transition of the respiratory state in a Cheyne-Stokes respiratory symptom in which apnea and respiratory states are repeated with transition of abnormal enhancement of sympathetic nerve.
4) A therapeutic system which comprises (1) an examination apparatus for use in selecting a patient for whom an oxygen therapy is effective among patients having a sleep respiratory disturbance, and/or use in ascertaining a therapeutic effect of the oxygen therapy, and (2) a supplying apparatus of an oxygen-enriched gas for respiration for the purpose of carrying out the oxygen therapy, wherein an output part for displaying or printing both of transition of respiratory airflow and transition of enhanced state of sympathetic nerve of the subject patient during sleeping is provided to the examination apparatus.
5) The therapeutic system according to the above item 4) wherein the supplying apparatus of an oxygen-enriched gas for respiration is constituted to allow flow rate of the oxygen-enriched gas for respiration to be regulatable within a predetermined range so that the flow rate becomes the amount prescribed on the basis of the result displayed or printed by the output part of the examination apparatus.
6) A method of selecting a patient for whom an oxygen therapy is effective among patients having a sleep respiratory disturbance which comprises: determining respiratory airflow and enhanced state of sympathetic nerve of a patient; and selecting a patient who exhibits both results that the determined state of sympathetic nerve is an enhanced state, and transition of enhanced state of sympathetic nerve is found in conjunction with transition of respiratory airflow.
7) The method of selecting a patient for whom an oxygen therapy is effective according to the above item 6) wherein the step of determining respiratory airflow of the patient detects a Cheyne-Stokes respiratory symptom in which apnea wave form and respiration wave form are repeated.
8) The method of selecting a patient for whom an oxygen therapy is effective according to the above item 7) wherein enhancement of sympathetic nerve occurs in conjunction with occurrence of the respiration wave form in a Cheyne-Stokes respiratory symptom of the patient.
9) The method of selecting a patient for whom an oxygen therapy is effective according to any one of the above items 6) to 8) wherein the step of determining the enhanced state of sympathetic nerve comprises determining electrocardiogram wave form of the patient, and the enhanced state of sympathetic nerve is analyzed based on the determined electrocardiogram wave form by a heart rate variability analytical procedure.
10) A method of selecting a patient for whom an oxygen therapy is effective among patients having a sleep respiratory disturbance which comprises: determining arterial oxygen saturation of a patient; determining respiratory airflow and enhanced state of sympathetic nerve of the patient; and selecting a patient who exhibits the results that an arterial oxygen saturation is not higher than a predetermined threshold value and the patient is in an enhanced state of sympathetic nerve, and transition of enhanced state of sympathetic nerve is found in conjunction with transition of respiratory airflow.
11) A therapeutic method for sleep respiratory disturbance which comprises: determining respiratory airflow and enhanced state of sympathetic nerve of a patient having a sleep respiratory disturbance; selecting a patient who exhibits both results that a state of sympathetic nerve is an enhanced state, and transition of enhanced state of sympathetic nerve is found in conjunction with transition of respiratory airflow; and administering oxygen to the patient.
12) The therapeutic method for sleep respiratory disturbance according to the above item 11) wherein the respiratory airflow of the patient exhibits a Cheyne-Stokes respiratory symptom in which apnea wave form and respiration wave form are repeated, and oxygen is administered to a patient in whom occurrence of enhancement of sympathetic nerve is found in conjunction with occurrence of respiration wave form in the Cheyne-Stokes respiratory symptom.
13) A therapeutic method for sleep respiratory disturbance which comprises: determining arterial oxygen saturation of a patient having a sleep respiratory disturbance; determining respiratory airflow and enhanced state of sympathetic nerve of the patient; selecting a patient who exhibits the results that an arterial oxygen saturation is not higher than a predetermined threshold value and the patient is in an enhanced state of sympathetic nerve, and transition of enhanced state of sympathetic nerve is found in conjunction with transition of respiratory airflow; and administering oxygen to the patient.

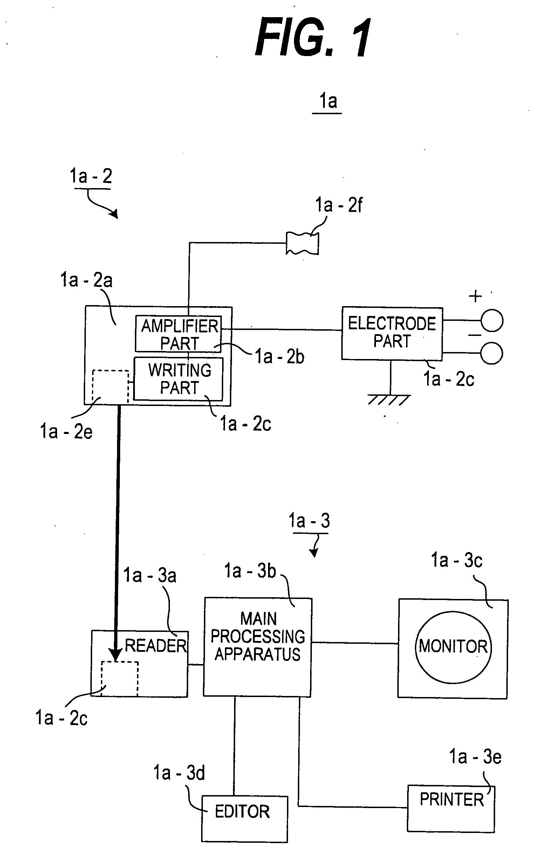

A therapeutic system for CHF (chronic heart failure) patients (hereinafter, may be merely referred to as "therapeutic system") which is a preferable Example according to an embodiment of the present invention will be explained below with reference to Fig. 1 to Fig. 4.

### Focused point which was responsible for practice of the constitution of this Example

The present inventor studied prior art constitutions which may relate to application of the oxygen therapy on CHF patients in detail. Consequently, understandings of problems in prior art as explained above were attained, and the following findings were obtained by numerous studies of applicable cases of the oxygen therapy on CHF patients.

More specifically, the findings include the following points that: (1) there may be a case in which a variety type of sleep respiratory disturbances are found in a CHF patient in addition to a definite Cheyne-Stokes respiratory symptom; (2) when such a sleep respiratory disturbance including a Cheyne-Stokes respiratory symptom is found, there may be a case in which enhancement of sympathetic nerve which is believed to result from this sleep respiratory disturbance is found; and (3) practice of an oxygen therapy is effective on a patient in whom the enhancement of sympathetic nerve is found, therefore, the method of selecting a patient, in whom a sleep respiratory disturbance and enhancement of sympathetic nerve are found, as a subject patient for carrying out an oxygen therapy is effective.

Constitution of the therapeutic system of this Example explained below was accomplished by focusing attention to each of the aforementioned findings, in particular, and the effect thereof will be clarified in description of specific constitution.

### Summary of constitution of therapeutic system

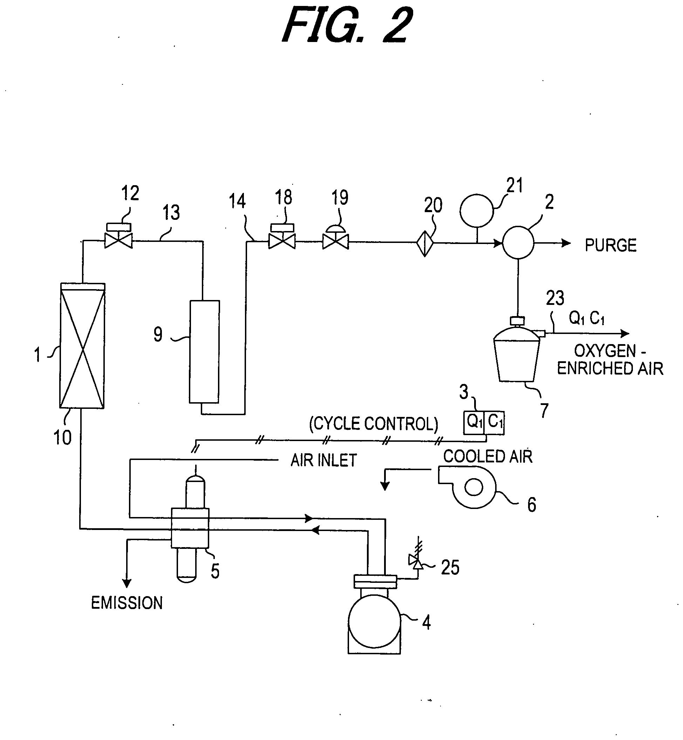

A therapeutic system for a patient having a Cheyne-Stokes respiratory symptom according to this Example which will be explained below has a biological information monitoring apparatus 1a illustrated in Fig. 1 and a supplying apparatus of a gas for respiration 2a illustrated in Fig. 2 as principal constitutions thereof.

An examination according to the procedures described below is performed on a patient for whom an oxygen therapy may be possibly effective, for example, a CHF patient, using the aforementioned biological information monitoring apparatus 1a to determine presence/absence of a sleep respiratory disturbance and enhancement of sympathetic nerve caused in the patient by this sleep respiratory disturbance, through performing examination according to the procedures described below, and on a patient exhibited a sleep respiratory disturbance and enhancement of sympathetic nerve is carried out an oxygen therapy using the supplying apparatus of a gas for respiration 2a based on a prescription and instruction of the medical service worker. Accordingly, a therapy has come to be allowed to be carried out by way of certainly and readily selecting a patient to whom an oxygen therapy should be carried out. Also, therapeutic effect of this oxygen therapy can be readily and certainly ascertained.

The biological information monitoring apparatus 1a may be used for selection of a patient for whom an oxygen therapy is effective by using alone or in combination with other constitution for a constitution other than the therapeutic systems 1a and 2a of this Example. Moreover, it may be also used for both purpose with ascertaining therapeutic effect of the oxygen therapy, or for single purpose of either one.

Furthermore, as a matter of course, constitution of a therapeutic system for patients having a Cheyne-Stokes respiratory symptom is also enabled through using other unit for supplying oxygen-enriched gas, for example, a unit for supplying gas oxygen with use of liquid oxygen, an oxygen cylinder for retaining compressed gas oxygen or the like in place of the supplying apparatus of a gas for respiration 2a.

Hereinafter, constitutions of therapeutic systems 1a and 2a of this Example will be explained in more detail.

### Constitution of biological information monitoring system

As illustrated in the constitution diagram shown in Fig. 1, constitution of the biological information monitoring apparatus la can be roughly separated into a biological information monitor la-2, and a biological information analysis apparatus 1a-3.

Moreover, the biological information monitor 1a-2 has an amplifier part la-2b provided inside of the main body 1a-2a, a writing part 1a-2c, an IC card 1a-2e provided inside of the main body 1a-2a in a detachable manner, an electrode part 1a-2d connected to the amplifier part 1a-2b via a lead wire and positioned outside of the main body 1a-2a, and a respiratory airflow sensor la-2f.

Additionally, a constitution to have a sensor other than the parts as described above may be also permitted.

The main body 1a-2a has a chassis structure constituted to be lightweight and compact, and can be attached to a lumbar region of a patient using a belt or the like. Consequently, the patient can easily move, for example, from the medical institution to home in the state having the biological information monitor la-2 attached, and in addition, each determination described below can be readily conducted while attaching to the patient during sleeping without burden to the patient.

Moreover, the amplifier part 1a-2b has a function to supply electric power to each sensor unit described above connected to this amplifier part 1a-2b via a lead wire, and to execute predetermined amplification through receiving a sensor signal from each sensor unit, and A/D conversion, thereby conducting output of the converted signal to the writing part 1a-2c.

The writing part 1a-2c has a function to record a digital signal entered from the amplifier part 1a-2b into the IC card 1a-2e.

The IC card 1a-2e is a recording medium which enables writing/reading of the digital signal. By constituting the IC card 1a-2e to be detachable from the main body 1a-2a, the IC card 1a-2e after determination and writing of the data of the patient can be removed from the biological information monitor main body 1a-2a. Thus, execution of analysis of the determined data is permitted through attaching it to the biological information analysis apparatus 1a-3 described later.

Also, the electrode part 1a-2d is a sensor for obtaining an electrocardiogram wave form of this patient through sticking each electrode of this electrode part 1a-2d to a predetermined site on the skin of the patient.

Further, the respiratory airflow sensor 1a-2f is a sensor for determining presence/absence and magnitude of the airflow caused by respiration of this patient through sticking to the vicinity of the nasal cavity of the patient, by measuring and detecting the temperature of the respiratory airflow and other temperature of the ambient air.

Next, the biological information analysis apparatus 1a-3 which is another great constituting unit of the biological information monitoring apparatus 1a will be explained. The biological information analysis apparatus 1a-3 has, as similarly illustrated in the constitution diagram shown in Fig. 1, a reader 1a-3a constituted so that the IC card 1a-2e is detachable, a main processing apparatus 1a-3b connected to this reader 1a-3a, a monitor 1a-3c connected to this main processing apparatus 1a-3b, an editor 1a-3d connected similarly to the main processing apparatus 1a-3b, and a printer 1a-3e connected similarly to the main processing apparatus 1a-3b.

The reader 1a-3a has a function to read out the recorded data from the attached IC card 1a-2e, and to conduct output to the main processing apparatus 1a-3b. In the biological information monitoring apparatus 1a of this Example, use of the IC card 1a-2e as a medium for recording the data determined from the patient is as described previously, but it can be also constituted as other candidate constitution such that a medium other than the IC card 1a-2e is used, for example, a flash memory, a magnetic optical disk, an optical disk, a magnetic tape, a magnetic diskette or the like, as a matter of fact.

Referring back to explanation of the constitution again, the main processing apparatus 1a-3b has a function to control and conduct output for the purpose of executing processings according to a predetermined processing procedure, and displaying, recording, transmitting and the like of the processed result through use of various biological information of this patient entered from the reader 1a-3a. Practical processing procedures will be described later.

The main processing apparatus 1a-3b uses, specifically, a constitution in which a dedicated program is installed in a frequently-used personal computer. As a matter of course, constitution of a dedicated hardware can be also put into practical use.

The editor 1a-3d has a constitution for use in editing the biological information entered from the reader 1a-3a into the main processing apparatus 1a-3b. For example, biological information obtained by determination for such a long period of time, 24 hours, is visually identified by a given medical service worker, and a zone to be subjected to data processing may be selected. To this end, the editor 1a-3d has an input unit such as pad or key board and a selection unit.

Moreover, the monitor 1a-3c is an apparatus having a display screen for showing to the medical service worker and the like through displaying determined value incorporated into and processed in the main processing apparatus la-3b of the biological information of the patient determined by various kinds of the sensor units, or through displaying results of analysis obtained via an analysis as separately demonstrated, which may be realized by a CRT display, a liquid crystal display or the like.

Further, the printer 1a-3e has a function to print information which can be displayed by the monitor 1a-3c, or a report summarizing these determination results and analysis results, depending on the input from the main processing apparatus 1a-3b, on a paper medium that is a printing medium.

### Constitution of supplying apparatus of a gas for respiration

Next, constitution of a supplying apparatus of a gas for respiration 2a will be explained with reference to Fig. 2.

The supplying apparatus of a gas for respiration 2a carried by the therapeutic systems 1a and 2a of this Example can execute supply of oxygen-enriched air through setting the flow rate to fall within a previously defined range by having the constitution described below. Therefore, an oxygen therapy can be carried out with a setting of the flow rate in accordance with a prescription and instruction of the oxygen therapy produced by the medical service worker based on the results of observation of each biological information of the patient using the biological information monitoring apparatus 1a.

Outline flow chart of a pressure fluctuation adsorption type oxygen concentrator as the supplying apparatus of a gas for respiration 2a is illustrated in Fig. 2. An adsorption cylinder 1 filled with an adsorbent such as molecular sieve 5A which is apt to adsorb nitrogen rather than oxygen is connected to a compressor 4 by a channel via a flow path switching valve 5, while a surge tank 9 which temporarily reserves oxygen-enriched air is connected to the adsorption cylinder 1 by a channel 13 via an automatic switching valve 12. In addition, a channel for air inlet having an air inlet muffler or the like and a channel for emission having an emission muffler or the like are provided to the flow path switching valve 5. In this Example, members of from the compressor 4 to the surge tank 9 constitute a generation unit of oxygen-enriched air.

Additionally, a channel 14 for an oxygen-enriched airflow pass having a pressure reducing valve 19 or the like is installed to the surge tank 9, and this channel 14 is provided with an orifice type flow rate regulator 2 as a flow rate control unit. An oxygen-enriched air supplying unit (not shown in the Figure) such as a nasal cannula or a mask for mouth is connected by a channel 23.

Furthermore, as a supplying amount setting unit 3, for example, an oxygen concentration selecting knob is provided, and the knob alters adsorption/desorption cycle time in conjunction with the switching valve 5 of the generation unit thereby increasing or decreasing oxygen concentration of the oxygen-enriched air from the adsorption cylinder 1. A pressure detecting unit 21 detects the pressure upstream of the orifice type flow rate regulator 2, and transmits the information to a unit for raising an alarm (not shown in the Figure) when an abnormality in the pressure arises. In addition, it is practically preferred that the channel 14 further has an automatic switching valve 18, a sterilizing filter 20, a humidifier 7 and the like, and is connected to a nasal cannula or a mask for mouth (oxygen-enriched air supplying unit) by the channel 23.

The supplying amount setting unit 3 can also be a flow rate regulator in terms of a display, however, in the invention, adsorption/desorption cycle time of the switching valve 5 is altered by the setting, which consequently leads to setting of the oxygen concentration.

Mode of operation of the supplying apparatus of an oxygen-enriched gas 2a shown in Fig. 2 involves: allowing adsorption of nitrogen through introducing pressurized air to the adsorption cylinder 1 via the valve 5 by a compressor 4 in the state of electromagnetic valves 12 and 18 open, as shown in Fig. 2; and retention of thus resulting oxygen-enriched air in the surge tank 9 via a channel 13.

The oxygen-enriched air retained in the surge tank 9 passes through a pressure reducing valve 19 and a sterilizing filter 20, and is regulated to have a predetermined flow rate by the orifice type flow rate regulator 2, moisturized by a humidifier 7 and supplied to a patient having a respiratory disease or the like through a nasal cannula or the like connected to the channel 23.

In the generation unit of the oxygen-enriched air, adsorption is continued for a predetermined time according to the oxygen concentration which had been set by the oxygen concentration selecting knob as the supplying amount setting unit 3, followed by closing of the valve 12 to switch the valve 5, thereby conducting desorption through reducing the pressure within the adsorption cylinder 1 using the compressor 4 as a vacuum pump. After conducting desorption for a predetermined time, the valve 5 is switched to introduce the pressurized air to the adsorption cylinder 1, and further, the valve 12 is opened to cause back flow of the oxygen-enriched air from the surge tank 9. Thus, the adsorption cylinder 1 is repressurized, and then the adsorption step is performed while subsequently introducing the pressurized air to the adsorption cylinder 1.

Oxygen-enriched air is obtained by repeating such steps of adsorption and desorption. When there is no alteration in oxygen concentration set by the supplying amount setting unit 3, adsorption/desorption cycle time of the oxygen concentrator is operated at a predetermined rate and for a predetermined time period to obtain the oxygen-enriched air.

Along with increase or decrease in the oxygen concentration set by the supplying amount setting unit 3, fundamentally, the aforementioned operation condition in the oxygen-enriched air generation unit, i.e., length of time period of the adsorption/desorption cycle time of the oxygen concentrator is altered with seldom alteration of the ratio of the adsorption cycle time and the desorption cycle time, or it is altered by alteration of ratio of the adsorption cycle time and the desorption cycle time in conjunction with alteration of the set oxygen concentration. Thus, oxygen-enriched air having a desired oxygen concentration is retained in the surge tank 9.

The oxygen-enriched air retained in the surge tank 9 passes through the channel 14, with the flow rate being regulated by the flow rate control unit 2, and is supplied to the patient from the oxygen-enriched air supplying unit 4 such as a nasal cannula or the like. When a preferable flow rate for the user is set by the flow rate control unit 2, this control unit may not be thereafter touched even though alteration of the supplying amount is caused.

By way of regulation of the cycle time, and by way of regulation of discharge amount in the discharge controlling valve provided within the flow pass 14 if further necessary, flow rate of the oxygen-enriched air supplied to the flow rate control unit 2 is kept substantially constant.

In other words, according to the supplying apparatus of a gas for respiration 2a of this Example, use of the knob for adjusting oxygen concentration of the supplying amount setting unit 3 enables adjustment of the oxygen concentration to fall within a previously decided range, and use of the flow rate control unit 2 allows supplying amount of the oxygen-enriched air (flow rate) to be regulated to fall within a previously decided range, similarly. Hence, operation while setting both or either one of these oxygen concentration and flow rate at a desired value falling within a previously decided range is enabled.

In cases of a supplying apparatus of a gas for respiration having a constitution in which the flow rate alone can be altered and set within a predetermined range while the oxygen concentration is fixed, provider of the supplying apparatus of a gas for respiration previously prepares multiple kinds of apparatuses to cover different oxygen concentrations. Thus, an apparatus having specifications suited for the prescription has come to be installed at this patient's home to be used in the oxygen therapy.

The constitution of the supplying apparatus of a gas for respiration is not limited to the mode explained above, but a therapeutic system with a constitution which is different from the aforementioned one may be accomplished using a known technical constitution.

### Summary of therapeutic procedure using the therapeutic system

Next, procedures for carrying out the therapy on a patient through selecting the patient for whom an oxygen therapy is effective using the therapeutic systems 1a and 2a having a constitution as explained above will be sequentially explained.

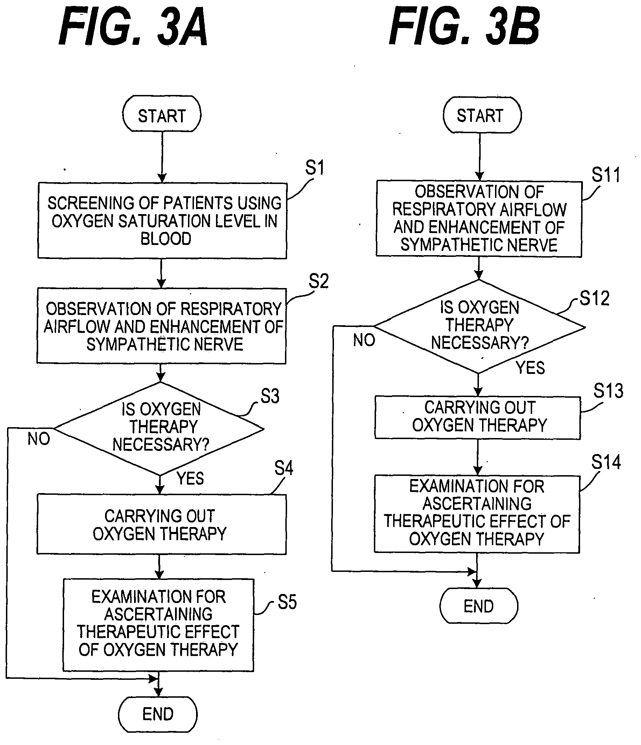

In the procedures for carrying out the therapy, as is illustrated in the flow chart shown in Fig. 3A, execution of screening of patients using arterial oxygen saturation for preliminarily selecting a candidate subject patient to be examined among CHF patients and other patients beforehand (step S1), subsequent observation of respiratory airflow and enhancement of sympathetic nerve using a biological information monitoring apparatus 1a as described above (step S2), decision for necessity to carry out an oxygen therapy by the medical service worker based on the results (step S3), carrying out an oxygen therapy using the supplying apparatus of a gas for respiration 2a when it was decided that an oxygen therapy should be carried out (step S4) are sequentially conducted.

When a predetermined therapeutic term is completed, an examination is performed to ascertain the therapeutic effect of the oxygen therapy using the biological information monitoring apparatus 1a (step S5). When the therapeutic effect could be ascertained, the series of therapeutic procedures are terminated, while when the therapeutic effect could not be ascertained or is insufficient, the oxygen therapy is carried out again through repeating the same therapy or through changing the condition setting so that grounds for the insufficient effect are avoided after specifying such grounds.

For reference, the procedures described above can be modified depending on the needs, for example, as shown in Fig. 3B, other each step can be also executed without conducting the screening of the patients using arterial oxygen saturation.

In the following description, each step will be explained according to the procedures shown in Fig. 3A.

### Screening of patients using arterial oxygen saturation (step S1)

Complication of a Cheyne-Stokes respiratory symptom is referred to as occurring in approximately 40% of CHF, as described above.

Further, it is known that arterial oxygen saturation of the patient is decreased lower than the normal level in a state of apnea or hypopnea in the Cheyne-Stokes respiratory symptom.
Also, decrease in arterial oxygen saturation is similarly found in a sleep respiratory disturbance other than a Cheyne-Stokes respiratory symptom, a complication symptom of CHF.

Thus, methods of conducting screening of patients are effective in which arterial oxygen saturation of a patient is continuously determined for a given determination period, for example, over a time period of 24 hours, and decides a patient as being suspected to have a sleep respiratory disturbance including a Cheyne-Stokes respiratory symptom when decrease in arterial oxygen saturation was found during the determination period.

In constitution for determining the arterial oxygen saturation, for example, use of "oxygen saturation monitor" (trade name: PULSOX®-M24, medical device manufacture approval number: 20900BZZ00154000) placed on the market by the present applicant may be also supposed (not shown in the Figure).

The PULSOX®-M24 is a pulse oximeter incorporating a 24 hours memory, which is of a wristwatch type and light weight, therefore, determination of the data during sleeping can be conducted without disturbing usual sleep state of the patient. Because data covering over a time period of 24 hours can be accumulated, recording for 2 to 3 nights is enabled. In addition, use of a dedicated analysis soft called DS-M allows ODI (SpO₂ frequency of decrease/hour), minimum SpO₂ value, total time during which the patient falls into a hypoxia state and the like to be calculated.

Subject patients for determination of the arterial oxygen saturation, i.e., population of patients for selecting a subject patient to whom decision for necessity to carry out an oxygen therapy should be made may be supposed to include chronic heart failure patients, patients suspected to be having a chronic heart failure, or patients suspected to have a complication of a Cheyne-Stokes respiratory symptom among chronic heart failure patients. Alternatively, the determination may be also supposed to be conducted on random subject patients in mass health screening or the like.

Because decrease in arterial oxygen saturation is also found in symptoms other than sleep respiratory disturbances including Cheyne-Stokes respiratory symptoms, it goes without mentioning that the determination step of arterial oxygen saturation merely screens patients suspected to have a sleep respiratory disturbance whatsoever.

### Examination on respiratory airflow and enhancement of sympathetic nerve (step S2)

Next, in order to perform examination for ascertaining: a sleep respiratory disturbance proven from respiratory airflow during sleep of the patient; and manifestation of enhancement of sympathetic nerve, the patient first comes to a medical institution as an outpatient, and the previously explained biological information monitor 1a-2 is attached on the whole to this patient at this medical institution.

Upon attachment, it is desired that an expert engineer ascertains with respect to, for example, proper attachment of the electrodes to the sites on the patient. When the ascertainment is completed, the patient goes home with keeping the biological information monitor 1a-2 attached, and collection of the biological information including sleeping time of the patient for, e.g., 24 hours is conducted.

When the determination is terminated, the patient visits to the medical institution again with keeping the biological information monitor 1a-2 attached, and then, the biological information monitor 1a-2 is removed and the IC card 1a-2e including the recorded determination data is recovered.

The recovery of the biological information monitor 1a-2 and the IC card 1a-2e may be also conducted by a method that is different from the above method. For example, the medical service worker (clinical laboratory technologist or the like) may visit the patient's home after the determination, and removal of the biological information monitor 1a-2 from the patient and recovery of the IC card 1a-2e can be also conducted while ascertaining the situation of attaching the sensors.

In addition, the determination during sleeping may be conducted not at home after the patient goes back with the biological information monitor 1a-2 attached, but while hospitalization in this medical institution. In this case, constitution of the biological information monitor 1a-2 is far simplified and lower cost than the equipment for PSG as described previously, and constant monitoring of the determination by an expert engineer is not also required, therefore, burdens to the medical institution can be significantly reduced in comparison with carrying out the PSG.

Analysis of the data recorded on the recovered IC card 1a-2e may be conducted using the previously explained biological information analysis apparatus 1a-3 in this medical institution, however, in many cases, it is sent from this medical institution to a specialized analysis center having the same biological information analysis apparatus 1a-3, and the analysis is conducted by an expert engineer in order to achieve the analysis efficiently.

Upon the analysis, the engineer selects a determination zone that presents a characteristic which is believed to be useful for diagnosis from the data in the entire determination zone using the editor 1a-3d, and the main processing apparatus 1a-3b produces a report including results of analysis of enhancement of sympathetic nerve of this patient using a heart rate variability analytical procedure (described later) with respect to the measurement value of the respiration level included in the selected determination zone, and electrocardiogram wave form determined in this zone. Specific contents described in the report will be demonstrated later.

Thus perfected report can be confirmed through displaying on the monitor 1a-3c, or may be printed on a paper medium that is a printing medium using a printer 1a-3e. Alternatively, the report is converted into electronic data which may be transmitted to the medical institution, or may be recorded on a recording medium to be sent to the medical institution. According to these procedures, output of the report can be conducted in a mode that is observable by the medical service worker in the medical institution.

The report printed on a paper medium is sent to the medical institution together with the IC card 1a-2e previously sent from the medical institution, and is used for diagnosis by a medical service worker such as a physician. The contents of the report can be altered, for example, by incorporating the determination results except for those described above.

### Analysis of modulation of autonomic nerve using a heart rate variability analytical procedure

A procedure for analyzing enhancement of sympathetic nerve of a patient using the aforementioned heart rate variability analytical procedure will be explained below.

The main processing apparatus 1a-3b carried by the therapeutic systems 1a and 2a of this Example conducts a heart rate variability analysis using the electrocardiogram wave form obtained from the patient. From the results of this analysis, transition of state of enhancement of sympathetic nerve of the patient is presented on a graph.

The heart rate variability analysis can be readily conducted through utilizing a constitution which has been reported as a known art. For example, JP-T-2001-505441 (the term "JP-T" as used herein means a published Japanese translation of a PCT application), page 7, lines 4 to 16 which refers to a constitution of "Implantable Medical Device Responsive to Heart Rate Variability Analysis" describes that "In one embodiment, a Holter monitor records R--R intervals while the patient exhibits normal or healthy heart rate variability. An algorithm based on mean and standard deviation then computes a single user value which is stored in permanent memory. This user value represents the patient's stress state during normal heart rate variability conditions. Thereafter, the patient wears a wrist detector which monitors the R--R intervals for discrete beat periods, for example 100 beats. Once a beat period is complete, the wrist detector and the algorithm are used to compute the patient's present user value or present stress state. This present user value is then compared to the permanently stored user value which was previously recorded under normal heart rate conditions. Theoretically, this comparison reveals deviations from normal heart rate variability which, in turn, are a measure of the patient's cardiac stress state. Large deviations between the two user values reflect large deviations in the autonomic nervous system balance between the sympathetic and parasympathetic activities.".

Further, the aforementioned patent document, page 17, lines 1 to 12 describes that "For example, a time domain analysis or a frequency domain analysis are two common ways researchers use to examine heart rate variability. In the time domain analysis, a graph typically displays the R--R intervals as the number of beats occurring during a specified time. As an example, ECG monitors may record and calculate heart rate variability. In the frequency domain analysis, a Fourier transform algorithm decomposes sequential R--R intervals into a sum of sinusoidal functions. A graph typically displays the result of this algorithm and shows the amplitude of the patient's heart rate fluctuations at different oscillation frequencies. The frequency domain analysis is particularly advantageous in some instances because certain frequency bands within the spectral analysis are associated with autonomic nervous system control of sinus node period. See J. Thomas Bigger, et. al, "Frequency Domain Measures of Heart Period Variability and Mortality After Myocardial Infarction" Circulation, Vol. 85 (1992), pp. 164-171.

Similarly, a home page provided on Internet to allow general public to be accessible <http://kansai.anesth.or.jp/kako/JSAKansai02abs/4.html> describes that "Autonomic activities in anesthesia induction and endotracheal intubation by way of slow administration of propofol used in combination with butorphanol were evaluated using an analysis apparatus of heart rate fluctuations Anemon-I, available from MCSA Medical Control SA (conducting a fractal analysis of R--R intervals, and digitalization of autonomic response).".

Based on these known documents and other known documents, the main processing apparatus 1a-3b can be constituted such that it conducts a heart rate variability analysis to produce a graph showing transition of patient's enhanced state of sympathetic nerve.

### Decision for necessity to carry out an oxygen therapy (step S3)

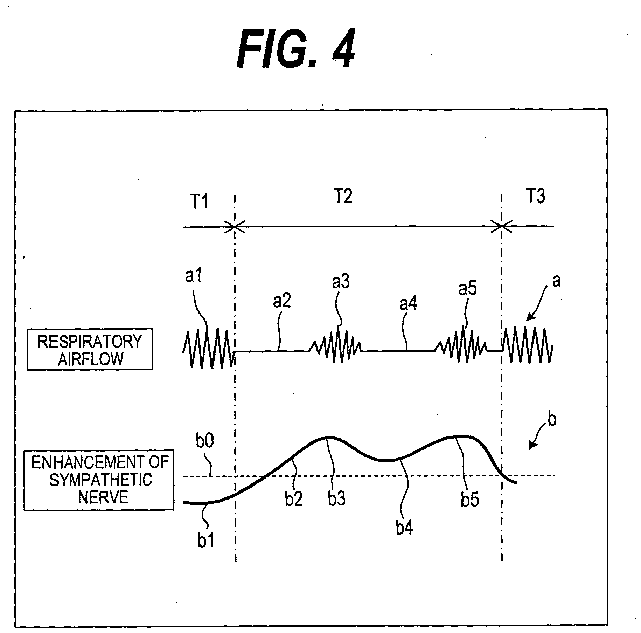

The report transmitted to the medical institution includes graphs showing each determination as illustrated in schematic view in Fig. 4. In Fig. 4, T1 and T3 represent a zone showing normal respiration; T2 represents a zone showing a Cheyne-Stokes respiratory symptom as a typical example of a sleep respiratory disturbance; "a" denotes a graph showing transition of a respiratory airflow level; "b" denotes a graph showing transition of enhancement of sympathetic nerve of the subject patient detected by the process of heart rate variability analysis.

Based on these contents in the report and other information, the medical service worker in the medical institution studies as to whether or not a sleep respiratory disturbance and enhancement of sympathetic nerve are found in this subject patient, and decision is made on whether or not an oxygen therapy should be carried out on the subject patient based on this study result.

The decision is made, for example, as follows. In illustrations in Fig. 4, referring to the respiratory airflow level "a" of this patient first, it can be found that constant level was kept in the period of T1 (a1), however, in the period of T2, abnormal respiration state was exhibited in which wave forms a2 and a4 showing apnea with lowered level, and wave forms a3 and a5 with repeating temporal increase and temporal decrease in the level are repeated. In addition, wave forms showing abnormal respiration other than Cheyne-Stokes respiratory symptoms illustrated in Fig. 4 may possibly exist.

Further, referring to the graph "b" showing transition of enhancement of sympathetic nerve, in the region b1 corresponding to the region a1 with normal respiratory airflow level, enhanced state of sympathetic nerve is found to be below the previously defined threshold b0. It is also found that in the regions b2 and b4 corresponding to the regions a2 and a4 showing apnea for the respiratory airflow level, enhancement of sympathetic nerve was temporarily increased to exceed the threshold b0, and in the regions a3 and a5 with repeating temporal increase and temporal decrease in the respiratory airflow level, the enhancement of sympathetic nerve reached to its peak, and turned into temporal decrease. Moreover, when the respiratory airflow level departs from the abnormal respiration zone T2 to enter the normal respiration zone T3, enhancement of sympathetic nerve is also decreased to be below the threshold b0.

From the observation results as described above, the medical service worker recognizes that: (1) a sleep respiratory disturbance is found in the subject patient; (2) enhancement of sympathetic nerve is found concurrent with development of this sleep respiratory disturbance; and (3) transition of state of enhancement of sympathetic nerve occurs in conjunction with transition of respiration airflow during development of a sleep respiratory disturbance.

From these findings, the medical service worker can definitely and obviously learn manifestation of enhancement of sympathetic nerve resulting from the sleep respiratory disturbance. Accordingly, effectiveness of an oxygen therapy for this sleep respiratory disturbance and enhancement of sympathetic nerve can be definitely and obviously learned, therefore, an instruction to carry out an oxygen therapy on this patient can be certainly conducted.

### Carrying out oxygen therapy (step S4)

Upon decision of carrying out an oxygen therapy, the medical service worker issues an instruction in which a prescription such as oxygen concentration, flow rate and the like of oxygen-enriched gas for respiration for this patient is described, and the supplying apparatus of a gas for respiration 2a is installed under the patient such as patient's home to carry out the oxygen therapy.

### Examination for ascertaining therapeutic effect of the oxygen therapy (step S5)

After a lapse of a given period following initiation of the oxygen therapy, the medical service worker instructs to perform an examination for ascertaining the therapeutic effect of this oxygen therapy. The examination may be executed by the procedure and the method that are the same as those in the examination of manifestation of the sleep respiratory disturbance and enhancement of sympathetic nerve as previously explained (step S2), and again, a report including the items as shown in Fig. 4 is produced. The medical service worker can not only ascertain whether or not the sleep respiratory disturbance itself was ameliorated by carrying out the oxygen therapy but also directly ascertain whether or not the enhancement of sympathetic nerve resulting from this sleep respiratory disturbance was ameliorated through studying this report. In other words, because amelioration of enhancement of sympathetic nerve that is one of target therapeutic items in an oxygen therapy can be directly, certainly and readily ascertained, great improving effect can be achieved in practices involved in medical services, according to the therapeutic systems 1a and 2a of this Example.

### Advantage of the Invention

As described in the foregoing, the present invention provides an examination apparatus and a therapeutic system for: certainly selecting a patient for whom an oxygen therapy is effective among, for example, CHF (chronic heart failure) patients; ascertaining a therapeutic effect of the oxygen therapy carried out on the selected patient without fail; and performing these operations without hospitalization using a simplified equipment at patient's home.

## Claims

1. An examination apparatus for use in selecting a patient for whom an oxygen therapy is effective among patients having a sleep respiratory disturbance, the apparatus comprising:
a sensor (1a - 2f) for detecting presence/absence or magnitude of respiratory airflow of the subject patient;
a unit (1a - 2d) for determining an electrocardiogram waveform of the subject patient, and an analysis unit (1a - 3) for analysing the enhanced state of sympathetic nerve based on the determined electrocardiogram waveform with a heart rate variability analytical procedure;
an analysis unit (1a - 3) for analysing synchronization of transition of the respiratory state into a Cheyne-Stokes respiratory symptom, in which apnea and respiratory states are repeated, with transition of enhancement of sympathetic nerve into an abnormal state and for producing graphs showing (A) said transition of respiratory airflow in conjunction with (B) said transition of enhanced state of sympathetic nerve, of the subject patient during sleeping; and
an output part (1a - 3c, 1a - 3e) for displaying or printing said graphs.

2. The examination apparatus according to claim 1 further comprising:
an IC card for recording the information of the respiratory airflow and the electrocardiogram wave of the subject patient, constituted to be detachable from the apparatus,
a reading unit for reading out the recorded information from the IC card, and
the analysis unit being operable to analyze the enhanced state of sympathetic nerve based on information read out from the reading unit.

3. A therapeutic system which comprises an examination apparatus (1a) according to claim 1 or 2, and a supplying apparatus of an oxygen-enriched gas for respiration for the purpose of carrying out the oxygen therapy.

4. The therapeutic system according to claim 3 wherein the supplying apparatus of an oxygen-enriched gas for respiration is constituted to allow flow rate of the oxygen-enriched gas for respiration to be regulatable within a predetermined range so that the flow rate becomes the amount prescribed on the basis of the result displayed or printed by the output part of the examination apparatus.

## Patentansprüche

1. Untersuchungsvorrichtung zur Verwendung bei der Auswahl eines Patienten, für den eine Sauerstofftherapie unter Patienten, die eine Schlafatemstörung aufweisen, wirksam ist, wobei die Vorrichtung umfasst:
einen Sensor (1a-2f) zum Detektieren des Vorhandenseins/Fehlens oder der Menge von Atemluftstrom des betreffenden Patienten;
eine Einheit (1a-2d) zum Bestimmen einer Elektrokardiogrammwellenform des betreffenden Patienten und eine Analyseeinheit (1a-3) zum Analysieren des erweiterten Zustands des Sympathikus basierend auf der bestimmten Elektrokardiogrammwellenform mit einem Analyseverfahren für Herzratenvariabilität;
eine Analyseeinheit (1a-3) zum Analysieren der Synchronisation des Übergangs des Atemzustands in ein Cheyne-Stokes-Atemsymptom, in dem Apnoe- und Atemzustände wiederholt werden, mit Übergang der Erweiterung des Sympathikus in einen abnormalen Zustand und zum Erzeugen von Graphen, die den Übergang des Atemluftstroms in Verbindung mit (B) dem Übergang des erweiterten Zustands des Sympathikus des betreffenden Patienten während des Schlafs zeigen (A); und
ein Ausgabeteil (1a-3c, 1a-3e) zum Anzeigen oder Drucken der Graphen.

2. Untersuchungsvorrichtung nach Anspruch 1 weiter umfassend:
eine IC-Karte zum Aufzeichnen der Information des Atemluftstroms und der Elektrokardiogrammwelle des betreffenden Patienten, die ausgebildet ist, um von der Vorrichtung abnehmbar zu sein,
eine Leseeinheit zum Auslesen der aufgezeichneten Information von der IC-Karte, und
die Analyseeinheit, die betrieben werden kann, um den erweiterten Zustand des Sympathikus basierend auf der Information zu analysieren, die von der Leseeinheit ausgelesen wurde.

3. Therapeutisches System, das eine Untersuchungsvorrichtung (1a) nach Anspruch 1 oder 2 und eine Zufuhrvorrichtung eines mit Sauerstoff angereicherten Gases zur Atmung zum Zweck der Ausführung der Sauerstofftherapie umfasst.

4. Therapeutisches System nach Anspruch 3, wobei die Zufuhrvorrichtung für ein mit Sauerstoff angereichertes Gas zur Atmung so ausgebildet ist, dass die Durchflussrate des mit Sauerstoff angereicherten Gases für die Atmung innerhalb eines vorbestimmten Bereichs regulierbar ist, so dass die Durchflussrate zu der Menge wird, die auf der Basis des Ergebnisses vorgeschrieben wird, das durch das Ausgabeteil der Untersuchungsvorrichtung angezeigt oder ausgedruckt wird.

## Revendications

1. Appareil d'examen pour une utilisation dans la sélection d'un patient pour lequel une oxygénothérapie est efficace parmi des patients ayant un trouble respiratoire du sommeil, l'appareil comprenant :
un capteur (la - 2f) pour détecter la présence/l'absence ou l'amplitude d'un flux d'air respiratoire du patient en question ;
une unité (la - 2d) pour déterminer une forme d'onde d'électrocardiogramme du patient en question, et une unité d'analyse (la - 3) pour analyser l'état amélioré du nerf sympathique sur la base de la forme d'onde d'électrocardiogramme déterminée avec une procédure d'analyse de variabilité de fréquence cardiaque ;
une unité d'analyse (la - 3) pour analyser la synchronisation d'un passage de l'état respiratoire à un symptôme respiratoire de Cheyne-Stokes, dans lequel une apnée et des états respiratoires sont répétés, avec un passage d'amélioration du nerf sympathique à un état anormal et pour produire des graphiques montrant (A) ledit passage du flux d'air respiratoire en relation avec (B) ledit passage d'un état amélioré du nerf sympathique, du patient en question pendant le sommeil ; et
une partie de sortie (la - 3c, la - 3e) pour afficher ou imprimer lesdits graphiques.

2. Appareil d'examen selon la revendication 1 comprenant en outre :
une carte IC pour enregistrer les informations sur le flux d'air respiratoire et l'onde d'électrocardiogramme du patient en question, conçue de façon à pouvoir être détachée de l'appareil,
une unité de lecture pour lire les informations enregistrées de la carte IC, et
l'unité d'analyse pouvant fonctionner de façon à analyser l'état amélioré du nerf sympathique sur la base des informations lues à partir de l'unité de lecture.

3. Système thérapeutique qui comprend un appareil d'examen (la) selon la revendication 1 ou 2, et un appareil d'alimentation d'un gaz enrichi en oxygène pour la respiration afin de réaliser l'oxygénothérapie.

4. Système thérapeutique selon la revendication 3 dans lequel l'appareil d'alimentation d'un gaz enrichi en oxygène pour la respiration est conçu de façon à permettre qu'un débit du gaz enrichi en oxygène pour la respiration puisse être réglé dans une plage prédéterminée de sorte que le débit devienne le niveau prescrit sur la base du résultat affiché ou imprimé par la partie de sortie de l'appareil d'examen.
